(19) Europäisches Patentamt / European Patent Office / Office européen des brevets

(11) **EP 3 806 770 B1**

(12) **EUROPEAN PATENT SPECIFICATION**

(45) Date of publication and mention
of the grant of the patent:
**26.07.2023 Bulletin 2023/30**

(21) Application number: **19731977.5**

(22) Date of filing: **18.06.2019**

(51) International Patent Classification (IPC):
***A61B 18/24*** (2006.01)

(52) Cooperative Patent Classification (CPC):
**A61B 18/24; A61B 5/0075; A61B 5/0084;
A61B 5/01; A61B 5/062; A61B 5/283;
A61B 5/4848; A61B 5/6843;** A61B 2017/00057;
A61B 2018/00166; A61B 2018/00351;
A61B 2018/00577; A61B 2018/00785;
A61B 2018/00982; A61B 2018/2211;        (Cont.)

(86) International application number:
**PCT/EP2019/065970**

(87) International publication number:
**WO 2019/243305 (26.12.2019 Gazette 2019/52)**

(54) **CATHETER WITH MERGED OPTICAL TISSUE EVALUATION AND LASER ABLATION**

KATHETER MIT FUSIONIERTER OPTISCHER GEWEBEBEURTEILUNG UND LASERABLATION

CATHÉTER AVEC ÉVALUATION DE TISSU OPTIQUE ET ABLATION LASER FUSIONNÉES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.06.2018 US 201862686159 P
13.06.2019 US 201916439946**

(43) Date of publication of application:
**21.04.2021 Bulletin 2021/16**

(73) Proprietor: **Medlumics S.L.
28760 Tres Cantos (ES)**

(72) Inventors:
• **LLORET SOLER, Juan
20835 Madrid (ES)**
• **RUBIO GUIVERNAU, José Luis
28050 Madrid (ES)**
• **GREENE, James L.
28760 Tres Cantos, Madrid (ES)**
• **JIMENEZ, Jorge H.
28760 Tres Cantos, Madrid (ES)**

(74) Representative: **DTS Patent- und Rechtsanwälte
Schnekenbühl und Partner mbB
Brienner Straße 1
80333 München (DE)**

(56) References cited:
**WO-A1-2016/187664      US-A- 5 290 275
US-A1- 2001 027 316     US-A1- 2015 209 105**

(52) Cooperative Patent Classification (CPC): (Cont.)
A61B 2090/064

**Description**

BACKGROUND

Field

Embodiments of the invention relate to a catheter with merged optical tissue evaluation and laser ablation.

Background

Ablation is a medical technique for producing tissue necrosis. It is used to help treat different pathologies including cancer, Barret's esophagus, or cardiac arrhythmias, among others. In some cases, various types of ablation may be utilized, such as cryogenic cooling for cryoablation, chemicals for chemical ablation, radiofrequency (RF) ablation, laser ablation, and the like. For radiofrequency (RF) ablation, the application of alternating current with an oscillating frequency above several hundreds of kHz avoids the stimulation of excitable tissue while delivering heat by means of the Joule's effect. The increase in tissue temperature produces denaturation of the biological molecules, including proteins such as collagen, myosin, or elastin. Traditionally, RF ablation is done by placing an external electrode on the patient's body, and applying an alternating potential to the tip of a catheter that is placed in contact with the tissue to be treated within the patient's body. In this context, a document WO 2016/187664 A1 should be mentioned. The ablation effect depends on a number of factors, including applied electrical power, quality of the electrical contact, local tissue properties, presence of blood flow close to the tissue surface, and the effect of irrigation. Because of the variability of these parameters, it is difficult to obtain consistent results.

BRIEF SUMMARY

Conventional ablation catheters and methods for RF ablation treatments are limited because of the challenges associated with aligning catheter electrodes with target tissues in order for accuracy during RF ablation procedures. In the embodiments presented herein, an ablation catheter for merged optical tissue evaluation and laser ablation is described.

The ablation catheter may provide a cost-effective solution to issues with RF ablation catheters that utilize complex electrical wiring for each electrode in the RF ablation catheter. In some embodiments, the ablation catheter includes a plurality of optical ports for both evaluating and ablating target tissue. By allowing laser ablation energy to be transmitted through the same optical ports that are used to perform tissue evaluation, the ablation catheter may use a single substrate that allows for focused evaluation of the same target tissue that is being ablated. In the embodiments presented herein, devices for performing optical tissue evaluation and laser ablation using catheters with a plurality of optical ports for transmitting exposure radiation beams and laser ablation energy to target tissue are described.

In an embodiment, a catheter system includes a catheter with a distal section, a proximal section, and a sheath coupled between the distal section and the proximal section, and a processing device. The distal section includes a plurality of optical ports and a holder configured to maintain the plurality of optical ports in a fixed spatial relationship. The plurality of optical ports are configured to transmit one or more beams of exposure radiation to a sample, receive one or beams of scattered radiation that have been reflected or scattered from the sample, and transmit laser ablation energy such that at least a portion of the sample is ablated. The processing device or the proximal section of the catheter includes a first optical source configured to generate a source beam of exposure radiation, and a second optical source configured to generate the laser ablation energy. The catheter system also includes a multiplexer configured to direct the one or more beams of exposure radiation from the source beam of radiation to the plurality of optical ports, combine the one or more beams of scattered radiation, and direct the laser ablation energy to at least one optical port of the plurality of optical ports. An example method for performing merged optical tissue evaluation and laser ablation is described. The method includes providing an ablation catheter, in which the ablation catheter includes a proximal end, a distal end with a plurality of optical ports, and a sheath coupled between the proximal end and the distal end. The method further includes transmitting one or more beams of exposure radiation via the plurality of optical ports to a sample near the distal end of the ablation catheter, receiving one or more beams of scattered or reflected radiation from the sample via the plurality of optical ports, and ablating at least a portion of the sample using laser ablation energy output from at least one optical port of the plurality of optical ports.

In another embodiment, a catheter system for performing merged optical tissue evaluation and laser ablation is described. The catheter system includes a catheter with a distal section, a proximal section, and a sheath coupled between the proximal section and the distal section, and a processing device. The distal section includes a plurality of optical ports configured to transmit one or more beams of exposure radiation to a sample, receive one or beams of scattered radiation that have been reflected or scattered from the sample, and transmit laser ablation energy such at least a portion of the sample is ablated. The processing device or the proximal section of the catheter includes a first optical source configured to generate a source beam of exposure radiation and a second optical source configured to generate the laser ablation energy. Further features and advantages, as well as the structure and operation of various embodiments, are described in detail below with reference to the accompanying drawings. It is noted that the specific embodiments described herein are not intended to be limiting. Such embodiments are presented herein for illustrative purposes only. Additional embodiments will be apparent to persons skilled in the relevant art(s) based on the teachings contained herein.

BRIEF DESCRIPTION OF THE DRAWINGS/FIGURES

The accompanying drawings, which are incorporated herein and form a part of the specification, illustrate embodiments of the present invention and, together with the description, further serve to explain the principles of the invention and to enable a person skilled in the pertinent art to make and use the invention.

FIG. 1 illustrates an example diagram of a catheter, according to embodiments of the present disclosure.
FIGs. 2A and 2B illustrate cross sections of a catheter, according to embodiments of the present disclosure.
FIGs. 3A-3G illustrate views of a distal end of a catheter, according to embodiments of the present disclosure.
FIGs. 4A and 4B illustrate additional views of a distal end of a catheter, according to embodiments of the present disclosure.
FIGs. 5A and 5B illustrate example block diagrams of the merged optical evaluation and laser ablation system of the catheter, according to embodiments of the present disclosure.
FIG. 6 illustrates an example graph showing blackbody spectrum at different wavelengths and temperatures.
FIG. 7 illustrates an example diagram of a layer stack for a silicon nitride waveguide, according to embodiments of the present disclosure.
FIG. 8 illustrates an example diagram of a device designed to direct a beam of radiation, according to embodiments of the present disclosure.
FIGs. 9A and 9B illustrate example graphs showing reflectivity as a function of wavelength and layer thickness, respectively, according to embodiments of the present disclosure.
FIG. 10 illustrates an example environment of a waveguide with an irrigation approach, according to embodiments of the present disclosure.
FIG. 11 illustrates an example graph showing focal plane positions of each wavelength at corresponding depth of focus values, according to embodiments of the present disclosure.
FIG. 12 illustrates an example method for performing merged optical tissue evaluation and laser ablation, according to embodiments of the present disclosure.

Embodiments of the present invention will be described with reference to the accompanying drawings.

DETAILED DESCRIPTION

Although specific configurations and arrangements are discussed, it should be understood that this is done for illustrative purposes only. A person skilled in the pertinent art will recognize that other configurations and arrangements can be used without departing from the scope of the present invention. It will be apparent to a person skilled in the pertinent art that this invention can also be employed in a variety of other applications.

It is noted that references in the specification to "one embodiment," "an embodiment," "an example embodiment," etc., indicate that the embodiment described may include a particular feature, structure, or characteristic, but every embodiment may not necessarily include the particular feature, structure, or characteristic. Moreover, such phrases do not necessarily refer to the same embodiment. Further, when a particular feature, structure or characteristic is described in connection with an embodiment, it would be within the knowledge of one skilled in the art to effect such feature, structure or characteristic in connection with other embodiments whether or not explicitly described.

As used herein, "laser energy" or "laser ablation energy" refers to light emitted through a process of optical amplification based on stimulated emission of electromagnetic radiation and refers to one or more beams of laser light that is generated by a laser source. It should be noted that although this application may refer specifically to cardiac ablation, the embodiments described herein may target other pathologies as well, along with additional energy sources for ablation. The principles of using laser ablation energy to treat other pathologies are similar, and therefore the techniques used to apply the laser ablation energy are similar.

Disclosed herein are embodiments of an ablation catheter for merged optical tissue evaluation and laser ablation in which the ablation catheter includes a plurality of optical ports for both evaluating and ablating target tissue. In some embodiments, the plurality of optical ports of the catheter may be configured to transmit beams of exposure radiation to a sample, receive one or more beams of scattered radiation that have been reflected or scattered from the sample, and transmit laser energy such that at least a portion of the sample is ablated. By utilizing the same optical ports for transmission of the optical evaluation signals and the laser ablation signals, the ablation catheter may provide focused evaluation of the same target tissue that is being ablated in a single substrate that allows for both modalities.

Herein, the terms "electromagnetic radiation," "light," and "beam of radiation" are all used to describe the same electromagnetic signals propagating through the various described elements and systems.

**Catheter Embodiments**

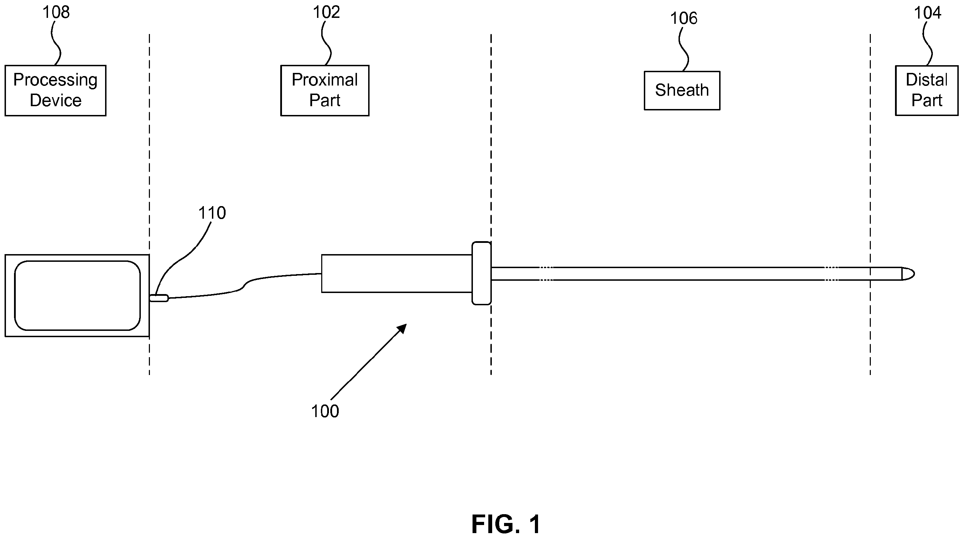

FIG. 1 illustrates a catheter 100 according to embodiments of the present disclosure. Catheter 100 includes a proximal section 102, a distal section 104, and a sheath 106 coupled between proximal section 102 and distal section 104. In an embodiment, sheath 106 includes one or more radiopaque markers for navigation purposes. In one embodiment, catheter 100 includes a communication interface 110 between catheter 100 and a processing device 108. Communication interface 110 may include one or more wires between processing device 108 and catheter 100. In other examples, communication interface 110 is an interface component that allows wireless communication, such as Bluetooth, WiFi, cellular, etc. Communication interface 110 may communicate with one or more transceiver elements located within either proximal section 102 or distal section 104 of catheter 100.

In an embodiment, sheath 106 and distal section 104 are disposable. As such, proximal section 102 may be reused by attaching a new sheath 106 and distal section 104 each time a new procedure is to be performed. In another embodiment, proximal section 102 is also disposable.

In some embodiments, various electrical and optical components such as a power supply, first and second optical sources, and interferometer elements are located in processing device 108. The electrical and optical signals from these components may be sent to proximal section 102 via communication interface 110. By housing these components in processing device 108, the whole of catheter 100 may be disposable.

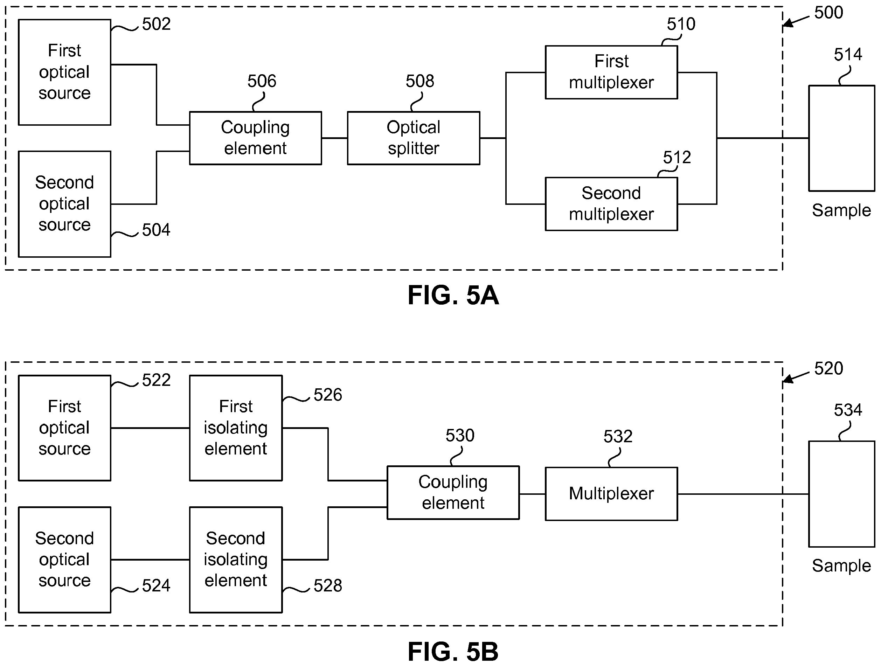

In other embodiments, proximal section 102 may house various electrical and optical components used in the operation of catheter 100. A first optical source may be included within proximal section 102 to generate a source beam of radiation for optical evaluation. The first optical source may include one or more laser diodes, superluminescent diodes (SLEDs) or light emitting diodes (LEDs). The beam of radiation generated by the optical source may have a wavelength within the infrared range. In one example, the beam of radiation has a central wavelength of 1.3 $\mu$m. The optical source may be designed to output a beam of radiation at only a single wavelength, or it may be a swept source and be designed to output a range of different wavelengths. The generated beam of radiation may be guided towards distal section 104 via the optical transmission medium connected between proximal section 102 and distal section 104 within sheath 106. Some examples of optical transmission media include single mode, polarization maintaining, or multimode optical fibers and integrated optical waveguides. In one embodiment, the electrical transmission medium and the optical transmission medium are provided by the same hybrid medium allowing for both electrical and optical signal propagation. Furthermore, proximal section 102 may include a second optical source, such as a laser energy source for tissue ablation. In some embodiments, the laser energy source may emit an ablation beam of laser energy at a wavelength of 980 nm or a wavelength of 1060 nm. The laser energy from the source in proximal section 102 may propagate down catheter 100 via an optical transmission medium connected between proximal section 102 and distal section 104 within sheath 106, and the laser energy may be output from the distal section 104 of catheter 100 to target tissue. For example, the laser energy from the source may produce an optical power of 5W to 12W that is applied to target tissue for 20-30 seconds to produce transmural lesions in heart tissue. In another example, the laser energy from the source may produce an optical power of 30W to 50W that is applied to target tissue for 60-90 seconds. Further details of how the first optical source for optical evaluation and second optical source for laser ablation are coupled together are discussed with reference to FIGs. 5A and 5B.

In an embodiment, proximal section 102 includes one or more components of an interferometer in order to perform low coherence interferometry (LCI) using the light generated from the first optical source. Due to the nature of interferometric data analysis, in an embodiment, the optical transmission medium used for guiding the light to and from distal end 104 does not affect the state and degree of light polarization. In another embodiment, the optical transmission medium affects the polarization in a constant and reversible way.

Proximal section 102 may include further interface elements with which a user of catheter 100 can control the operation of catheter 100. For example, proximal section 102 may include a deflection control mechanism that controls a deflection angle of distal section 104. The deflection control mechanism may require a mechanical movement of an element on proximal section 102, or the deflection control mechanism may use electrical connections to control the movement of distal section 104. Proximal section 102 may include various buttons or switches that allow a user to control when laser energy is applied at distal end 104, or when the beams of radiation are transmitted from distal end 104, allowing for the acquisition of optical data. In some examples, these buttons or switches are located at a separate user interface coupled to processing device 108.

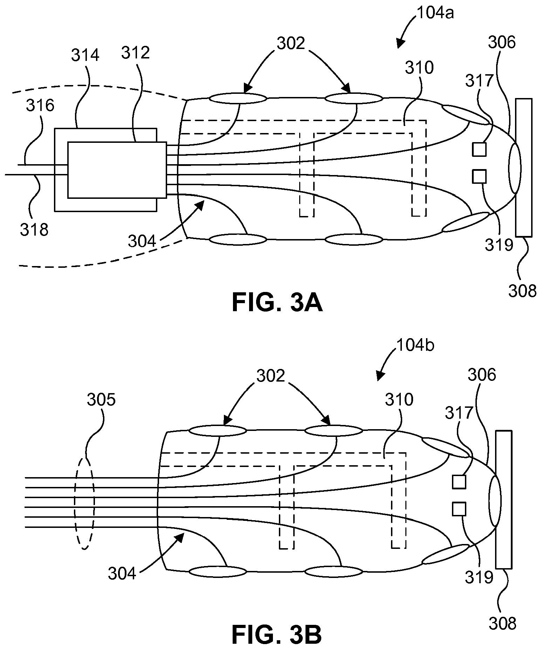

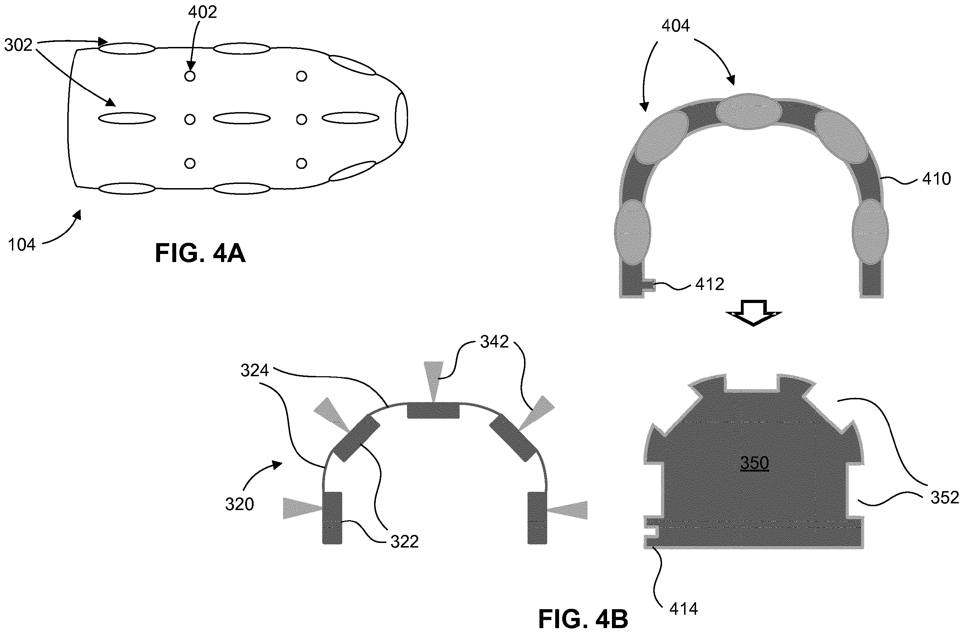

Distal section 104 includes a plurality of optical view ports, which will be described further detail below. In an embodiment, one or more of the optical view ports are machined into the outer body of distal section 104. For example, one or more of the optical view ports of the outer body of distal section 104 may be produced by laser drilling, 3D printing, stereolithography (SLA), fused deposition modeling (FDM), selective laser sintering (SLS), and/or other techniques. The optical view ports are distributed over the outside of distal section 104, resulting in a plurality of distinct viewing directions. The optical view ports also allow for a plurality of directions in which laser energy may be directed for tissue ablation through one or more of the optical view ports. In an embodiment, each of the plurality of viewing directions are substantially non-coplanar. The optical view ports may also be designed with irrigation functionality to cool distal section 104 and surrounding tissue during ablation. Further details on the design of distal section 104 are discussed with reference to FIGs. 3A-3G and FIGs. 4A-4B.

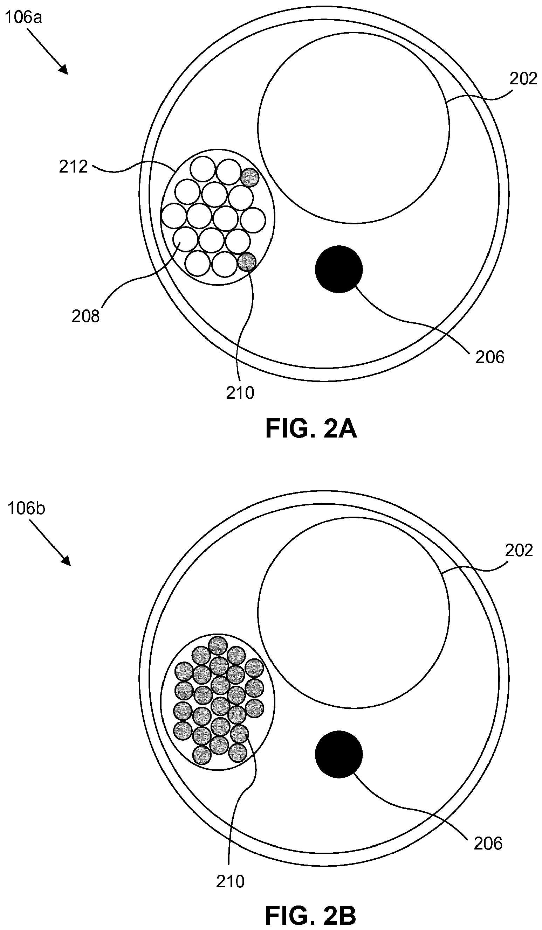

FIGs. 2A and 2B illustrate cross-section views of sheath 106, according to embodiments of the present disclosure. Sheath 106 may include all of the elements interconnecting proximal section 102 with distal section 104. Sheath 106a illustrates an embodiment that houses an irrigation channel 202, deflection mechanism 206, electrical connections 208, and optical transmission media 210. FIG. 2A illustrates a protective cover 212 wrapped around both electrical connections 208 and optical transmission media 210. Electrical connections 208 may be used to provide signals to optical modulating components located in distal section 104. One or more optical transmission media 210 guide light generated from the optical source (exposure light) towards distal section 104, while another subset of optical transmission media 210 guides light returning from distal section 104 (scattered or reflected light) back to proximal section 102. In another example, the same one or more optical transmission media 210 guides light in both directions. In some embodiments, one or more optical transmission media 210 may also be utilized to propagate the ablation laser energy (e.g., generated from a laser energy source) to distal section 104 of catheter 100.

Irrigation channel 202 may be a hollow tube used to guide cooling fluid towards distal section 104. Irrigation channel 202 may include heating and/or cooling elements disposed along the channel to affect the temperature of the fluid. In another embodiment, irrigation channel 202 may also be used as an avenue for drawing fluid surrounding distal section 104 back towards proximal section 102.

Deflection mechanism 206 may include electrical or mechanical elements designed to provide a signal to distal section 104 in order to change a deflection angle of distal section 104. The deflection system enables guidance of distal section 104 by actuating a mechanical control placed in proximal section 102, according to an embodiment. This system may be based on a series of aligned and uniformly spaced cutouts in sheath 106 aimed at providing unidirectional deflection of distal section 104, in combination with a wire which connects the deflection mechanism control in proximal section 102 with the catheter tip at distal section 104. In this way, a certain movement of the proximal section may be projected to the distal section. Other embodiments involving the combination of several control wires attached to the catheter tip may enable the deflection of the catheter tip along different directions.

FIG. 2B illustrates a cross-section of sheath 106b. Sheath 106b depicts an embodiment having most of the same elements as sheath 106a from FIG. 2A, except that there are no electrical connections 208. Sheath 106b may be used in situations where modulation (e.g., multiplexing) of the generated beam of radiation is performed in proximal section 102 or in processing device 108. In some embodiments, sheath 106b may include electrical connections for an electrode in distal section 104 for measuring electrocardiograms of tissue.

FIGs. 3A-3G illustrate views within distal section 104, according to embodiments of the present disclosure. For example, FIG. 3A illustrates distal section 104a having a plurality of view ports 302, a plurality of waveguides 304, and one or more irrigation channels 310 located substantially at a tip of distal section 104a. Plurality of view ports 302 may be arranged around the outside of distal section 104a in any pattern to achieve various views of a sample 308. Laser energy may be directed to at least one view port 302 of the plurality of view ports 302 to ablate a portion of sample 308. In an embodiment, waveguides 304 may be any type of waveguiding structures, such as waveguides defined within an optical integrated circuit. In another embodiment, waveguides 304 may be waveguiding structures defined upon a flexible substrate. In yet another embodiment, waveguides 304 may be optical fibers. A multiplexing unit 312 may also be defined upon the same flexible substrate that includes the waveguiding structures.

In FIGs 3A and 3B, waveguides 304 are used at each of plurality of view ports 302 to both transmit and receive light through each of plurality of view ports 302. Exposure light is transmitted through view ports 302 away from distal section 104a and onto sample 308, while light that is scattered or reflected by sample 308 is received through view ports 302. Each view port of plurality of view ports 302 may include more than one optical fiber, for example, a fiber bundle. Light generated from the first optical source (e.g., for tissue evaluation) within proximal section 102 or processing device 108 may be split amongst each of the view ports 302 using multiplexing unit 312. Alternatively, multiplexing unit 312 may select one of the plurality of view ports 302 for light to travel either to or from.

Additionally, laser energy generated from the second optical source (e.g., for laser ablation) within proximal section 102 or processing device 108 may be directed to one or more view ports 302 using the same multiplexing unit 312 or a different multiplexing unit (not shown) to select at least one of the plurality of view ports 302 for targeted ablation. Multiplexing unit 312 receives an input beam of radiation and a laser beam via optical transmission line 316. Optical transmission line 316 may include any number of optical transmission elements (e.g., optical fibers), and may be similar to optical transmission media 210 of FIGs. 2A and 2B. Electrical wires 318 may be included to carry control signals to multiplexing unit 312 from proximal section 102 of catheter 100 or from processing device 108.

Multiplexing unit 312 may include associated electronics 314 that provide control signals to various modulating elements of multiplexing unit 312. Multiplexing unit 312 may use any multiplexing method that allows for the separation of contributions from the light collected by various view ports 302, as well as separation of the light for optical tissue evaluation and the light for laser ablation. One such multiplexing method is time-domain multiplexing, in which multiplexing unit 312 switches between different output waveguides in a controlled manner, so that at a given time only one of the associated view ports 302 is active. Another suitable multiplexing method is frequency-domain multiplexing, in which light traversing each of view ports 302 is modulated in such a way that the time-frequency behavior of signals corresponding to different view ports 302 can be differentiated by a processing device (e.g., processing device 108). Coherence-domain multiplexing may also be used in multiplexing unit 312, by introducing a different group delay to the light traversing each view port 302, so that the signals corresponding to different view ports 302 appear at different coherence positions and can be therefore differentiated by a processing device (e.g., processing device 108). These methods are non-exclusive and can be combined in order to find the best design compromise. Some of the multiplexing methods, like coherence-domain multiplexing, do not require any electrical actuation of multiplexing unit 312. Thus, in an embodiment, implementations based on coherence-domain multiplexing do not require electrical transmission media for control signals.

In one embodiment, multiplexing unit 312 is produced on a silicon nitride photonics chip using a network of thermo-electric optical switches. Other suitable materials for use in multiplexing unit 312 include silicon dioxide, oxinitride, lithium niobate, III-V semiconductor materials, silicon on insulator (SOI), gallium arsenide (GaAs), silicon carbide or optical grade polymers, and the like. Other modulation effects to support the optical switching operation include the electro-optic effect, charge carrier density effects, photo-mechanical effects, liquid crystal based refractive index modulation, etc. The multiplexing function may also be obtained through microelectromechanical (MEMS) devices in as far as miniaturization and packaging constraints can be met. The connections between electrical wires 318 and multiplexing unit 312 may be achieved via individual wire-bonding or soldering, or through an intermediate substrate that allows for flip-chip assembly in an individual or batch process. In an embodiment, this intermediate substrate is flexible.

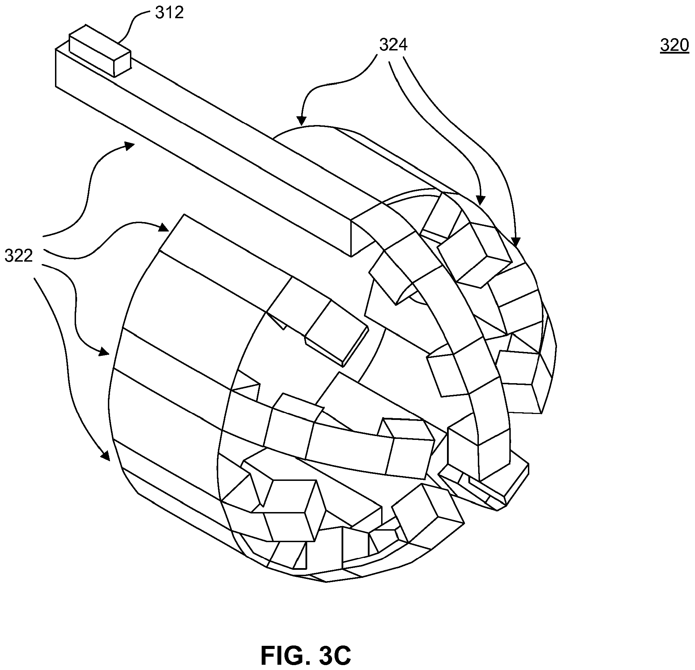

In an embodiment, multiplexing unit 312 is fabricated upon a flexible substrate. A process for forming the optical elements upon a flexible substrate includes a substrate transfer post-processing step applied to Silicon on Insulator (SOI) chips or wafers, as described in more detail in U.S. Pat. No. 9062960. In an embodiment, the resulting flexible device is thinner (<350 $\mu$m) than the starting thickness (500-700 $\mu$m). Multiplexing unit 312 may be implemented by an optical integrated chip that is partly flexible. Plurality of waveguides 304 (e.g., optical fibers) are suitably flexible in order to reach the various view ports 302 arranged round distal section 104a, according to an embodiment. As illustrated in FIG. 3C - 3G, the optical integrated chip may be formed from a series of interconnected rigid sections joined by flexible sections. Associated electronics 314 may be attached to either the bottom side or top side of an integrated chip that includes multiplexing unit 312. In another embodiment, both multiplexing unit 312 and associated electronics 314 are disposed upon a flexible substrate. In one example, the flexible substrate having both multiplexing unit 312 and associated electronics 314 is rolled in a cylindrical shape to fit within distal section 104a of catheter 100.

As shown in FIG 3A, distal section 104a may include one or more irrigation channels 310 to deliver fluid to a plurality of holes (not shown) on the outside of distal section 104a. The fluid delivered via irrigation channels 310 may be used for cooling during the ablation procedure. In other embodiments, irrigation channels 310 may be designed to deliver therapeutic fluids to sample 308.

Distal section 104a may also include a force sensor 317. In an embodiment, force sensor 317 is designed to measure a force applied to distal section 104a during operation along one or more reference axes. Force sensor 317 may include a rigid element coming from the sheath (e.g. a rigid wire) mechanically connected to a part of the sensor. The general assembly of the catheter and any mechanical fixation element acting between distal section 104a and the sheath must ensure sufficient stress transfer to force sensor 317. In another embodiment, force sensor 317 may be a pressure sensor based on, for example, a strain gauge.

Force sensor 317 may have its readout element defined in the same substrate as multiplexing unit 312, according to an embodiment. The read-out principle may be based on an interferometric analysis of distance change associated to strain, on an spectral analysis of resonant-type devices, on a piezo-electric device, on a capacitance measurement, or based on an electromagnetic measurement. According to an embodiment, the signals generated from force sensor 317 propagate through additional cables and/or optical transmission media running through sheath 106. Alternatively, the signals may propagate through the same electrical and optical paths used for multiplexing unit 312 and its associated electronics 314. In the latter case, the multiplexed optical path and force sensor 317 data path may be separated through a suitable signal multiplexing technique. Additionally, if irrigation channels 310 are perfused at a low and constant flow, the pressure may be measured indirectly by adding a pressure transducer in proximal section 102 of catheter 100.

In an embodiment, a temperature sensor 319 may be included in distal section 104a, measuring the temperature substantially at the tip of the catheter during operation. Although FIGs. 3A and 3B illustrate only one temperature sensor 319, there may be any number of temperature sensors 319 in distal section 104. Temperature sensor 319 may be a thermo-couple, an element with a known resistive dependence on temperature, an element where an optical parameter changes with temperature, or any other type of temperature sensor. Temperature sensor 319 may be included as an element defined in the same substrate as multiplexing unit 312. According to an embodiment, the signals generated from temperature sensor 319 propagate through additional cables and/or optical transmission media running through sheath 106, or through the same electrical and optical paths used for multiplexing unit 312 and its associated electronics 314. In the latter case, the multiplexed optical path and temperature sensor 319 data paths may be separated through a suitable signal multiplexing technique.

In some embodiments, distal section 104 may include one or more electrodes for measuring electrocardiograms of tissue and/or one or more magnetic sensors for allowing navigation of the catheter. For example, one or more magnetic sensors of the catheter may be combined with externally-generated magnetic field generators for navigating the catheter with magnetic fields.

FIG. 3B illustrates another embodiment of the distal section, depicted as distal section 104b. Distal section 104b includes many of the same elements as those described in distal section 104a. However, distal section 104b does not include multiplexing unit 312 and associated electronics 314. A bundle of fibers 305 is used to provide light to the plurality of waveguides 304 within distal section 104b. In a catheter embodiment using distal section 104b, a multiplexing unit may be located within proximal section 102 or external to catheter 100 (such as, for example, with processing device 108).

In either embodiment of distal section 104 illustrated in FIGs. 3A and 3B, the plurality of view ports 302 may include one or more lenses and/or mirrors or similar optical elements designed to focus the light traversing any of view ports 302. The material used within each view port 302 is substantially transparent to a range of wavelengths of light used for optical interrogation and a range of wavelengths of light used for laser ablation, according to an embodiment. For example, the material used within each view port 302 may be substantially transparent at 1.3 $\mu$m for optical tissue evaluation and at 980 nm or 1060 nm for laser ablation. The optical element may be coated with an antireflective layer to minimize optical losses. The mirrors may be locally produced through the selective evaporation of a metal layer through a mask on the surfaces to be made reflective, and may be flat or provide a focusing function. In some embodiments, total internal reflection (TIR) mirrors which do not require metal coating may also be used to redirect and/or focus light. The body of distal section 104 may be formed using injection molded plastic, and designed to support the packaging of multiplexing unit 312. In an embodiment, the optical element used at the plurality of view ports 302 includes gradient index lenses and/or lenses with tapered tips.

In an embodiment, one or more of the plurality of view ports 302 includes a scanning element (not shown) that allows for one or more beams exiting through view port 302 (e.g., one or more beams of the exposure radiation and the laser ablation energy) to be scanned in a given direction. The scanning element may include a microelectromechanical system (MEMS) component, or use electro-optical modulators to steer the exit angle of the beam of radiation from an associated view port. Further details and examples regarding the scanning of the beams of radiation may be found in U.S. Pat. No. 9354040. In some embodiments, the scanning of the exposure radiation allows for the collection of optical coherence tomography (OCT) images of a sample.

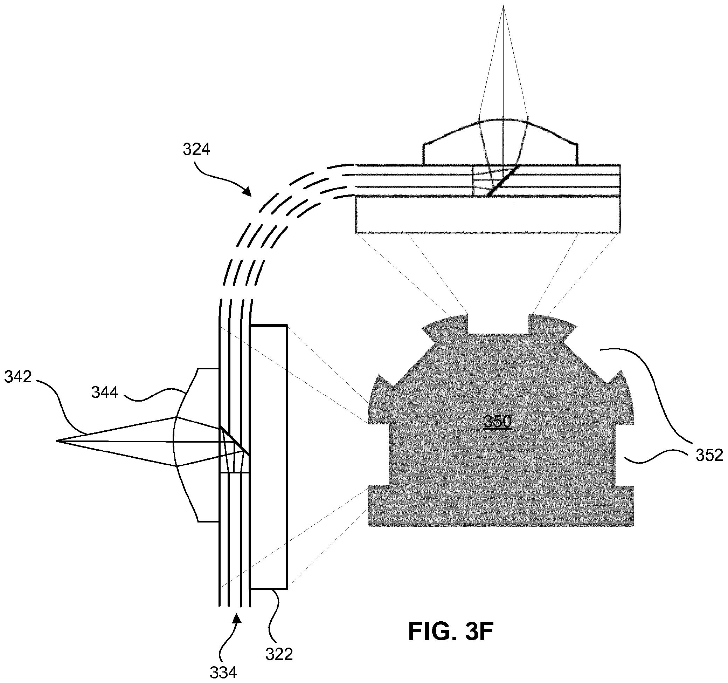

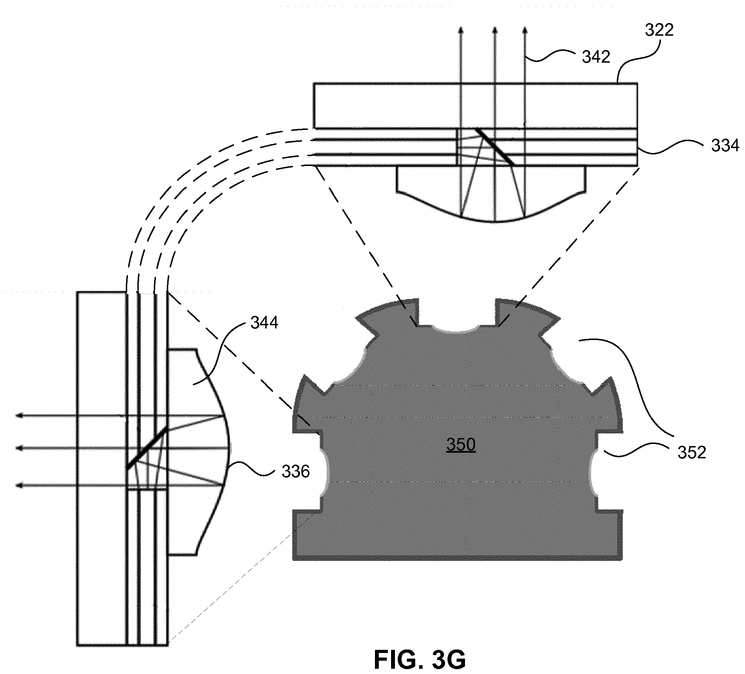

FIGs. 3C-3G illustrate additional embodiments of distal section 104. These embodiments include many of the same elements as those described in FIGs 3A and 3B unless otherwise noted. Each of the arrangements depicted in FIGs. 3C-3G illustrate distal sections that include a photonic integrated circuit having rigid sections joined by flexible sections. As illustrated in FIG. 3C, a single substrate 320 may be used as a base for all rigid beam input/output sections 322. Each rigid beam input/output section 322 is connected to at least one thin flexible section 324. Beam input/output sections 322 may provide optical ports and are optically and mechanically interconnected by flexible sections 324 to form the tip of distal section 104. Substrate 320 thus has a plurality of integral branches, with each branch including rigid beam input/output sections 322 interconnected by flexible sections 324. The branches bend around the tip of the distal end at the flexible sections 324. In this way, different shapes and arrangements can be accommodated. A holder (illustrated in FIGs. 3F and 3G) may be used to fix the spatial relationship of the branches and corresponding rigid beam input/output sections 322.

Flexible waveguides may extend across the flexible sections to optically connect optical ports to multiplexer 312. The flexible sections may be formed by partial removal of the substrate material to thin the substrate. A layer of polyimide may be added to reinforce the thinned portion. Multiplexer 312 may be formed on a rigid section as shown in FIG. 3C or implemented across the rigid and flexible sections. Though it is easier to form beam optical ports on the rigid sections, beam optical ports may be formed on flexible sections as well. In an embodiment, optical couplers, e.g. 2 x 1 or 2 x 2, may be appropriately dispersed across the rigid and/or flexible sections to form part of multiplexer 312.

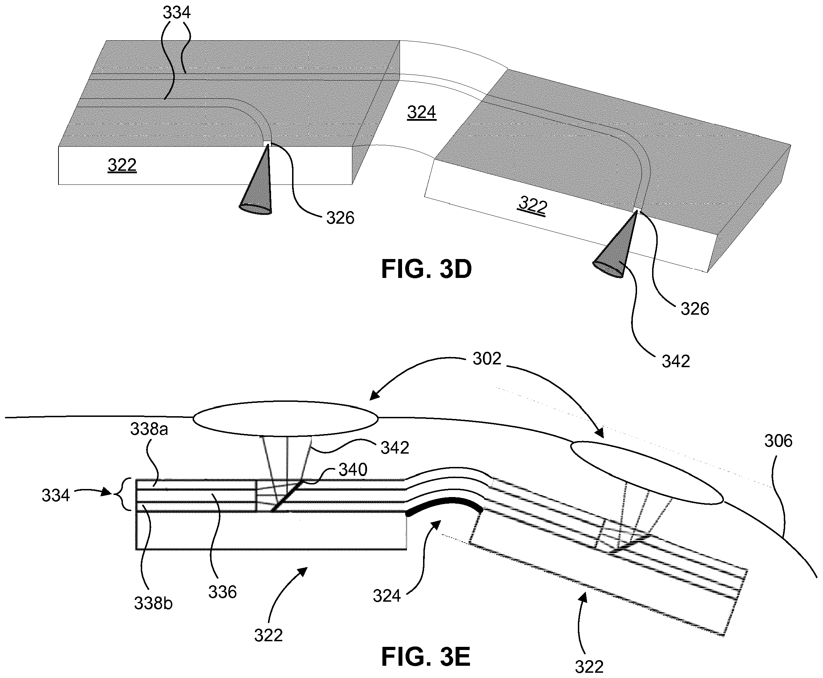

Beam input/output sections 322 may output focused or unfocused beams from optical ports. In addition, the beam may exit in the plane of a beam input/output section 322 (as shown in FIG. 3D) or may exit at an oblique angle to the plane of the beam input/output section 322, such as orthogonal to the plane (as shown in FIGs. 3E - 3G). Radiation may then be received from the sample along the same optical path. FIGs. 3E - 3G illustrate various embodiments of a device designed to direct a beam of radiation. Further details and alternative arrangements may be found in U.S. Pat. No. 9690093. FIG. 3D shows two beam input/output sections 322 optically and mechanically coupled by flexible section 324. Waveguides 334 run along the plane of the substrate to optical ports 326, in which the optical ports 326 are configured to direct beams of exposure radiation and laser ablation energy to target tissue. Optical ports 326 direct beams of radiation 342 substantially parallel to the plane of propagation along each respective beam input/output section 322. Optical ports 326 may also direct exposure radiation and laser energy without requiring alignment between optical ports 326 and with view ports 302 to pass light to the area of interest. Beam input/output sections 322 are not limited to a single optical port, but may instead have a plurality of optical ports.

FIGs. 3E - 3G illustrate the concept of directing a beam of radiation at an angle that is substantially perpendicular to a surface of the substrate. However, the embodiments differ in the placement and formation of certain elements. For example, FIG. 3E illustrates a substrate 320 formed by a plurality of beam input/output sections 322 mechanically and optically coupled by flexible section 324. The area below the flexible section has been removed to impart flexibility. One or more waveguides 334 are formed on each of the beam input/output sections 322. Waveguides 334 may extend across flexible section 324 to permit optical coupling between optical ports. Waveguide 334 includes a core layer 336 surrounded by cladding layers 338a and 338b. A reflector 340 is formed in-plane with waveguide 334 and is designed to reflect a beam of radiation 342 towards view port 302.

Substrate 320 may be any suitable material that allows for surface and/or bulk micromachining patterning steps to be performed. In one example, substrate 320 is a crystalline material such as silicon dioxide, silicon, gallium arsenide, indium phosphide, or the like. In other examples, substrate 320 is amorphous such as glass or polysilicon. Core layer 336 of waveguide 334 may comprise a material having a higher refractive index than cladding layers 338a and 338b in order to confine a beam of radiation propagating through waveguide 334. For example, core layer 336 may comprise silicon nitride ($Si_3N_4$). Waveguide 334 may have a crystalline structure or be a polymer. For example, waveguide 334 may be formed from one or more materials that are transparent at the wavelengths utilized for tissue evaluation and laser ablation and capable of implementing phase shifting mechanisms and handling high optical intensities with negligible non-linear effects. In one example, cladding layers 338a and 338b are silicon dioxide, substrate 320 is silicon, and core layer 336 is silicon oxide. Waveguide 334 may be a strip waveguide, ridge waveguide, an optical fiber laid across the surface of substrate 320 or any other type.

Reflector 340 may be formed from etching the layers that form waveguide 334, according to an embodiment. A wet anisotropic etchant (e.g., tetramethyl ammonium hydroxide (TMAH) and/or potassium hydroxide (KOH)) may be used to strip away the material along the crystal planes to form the surface of reflector 340. The surface may be further smoothed via thermal oxidation of silicon and oxide removal process by quickly exposing reflector 340 to another chemical etchant such as hydrofluoric acid (HF). Dry etching techniques may be employed as well for creating the angled surface of reflector 340. For example, reactive ion etching (RIE) using a grey-scale type mask to produce photoresist at varying heights can be used to produce non-planar structures. Reflector 340 is placed a short distance from an end of waveguide 334, according to an embodiment. This distance cannot be too large, or else the beam of radiation exiting from waveguide 334 will spread too far and undesirable optical losses will occur. In this embodiment, both reflector 340 and waveguide 334 are patterned in-plane on a first surface of substrate 320. Reflector 340 may be designed to have a surface that is angled. For example, reflector 340 may have a surface that is angled at a substantially 45 degree angle with respect to the first surface of substrate 320. This angle causes the beam of radiation to be directed at an angle that is substantially perpendicular to the surface of substrate 320. In another example, reflector 340 has a surface that is angled at a substantially 54.74 degree angle with respect to the first surface of substrate 320. In the embodiment illustrated in FIG. 3E, the light is reflected up and away from rigid beam input/output sections 322 towards view port 302. View port 302 may include a focusing optical element, such as a lens, to focus the divergent beam of radiation.

FIG. 3F shows an alternative arrangement that does not need an additional focusing element. Instead, an optical element 344 is disposed over waveguide 334 and over a top surface of rigid beam input/output section 322, according to an embodiment. In this embodiment, optical element 344 is a lens. The lens may be designed to focus beam of radiation 342 or to collimate beam of radiation 342. Optical element 344 may be manufactured using nano-imprint lithography or standard lithography etching using a grey-scale mask. Thermal reflow of a transparent polymer may also be used to form the curved lens shape. Optical element 344 may be fabricated using RIE directly in substrate 320. The advantage of using RIE may be realized when the substrate material has a high refractive index (e.g., material such as silicon, InP, etc.), thus the performance of the lens depends much less on the refractive index of the surrounding media. The curvature and position of the focusing surface of the lens may be adjusted so that the focal point and focal distance of the lens achieve the desired collimating or focusing performance. In one example, an intermediate polymer layer is introduced between optical element 344 and waveguide 334 in order to set a lens working distance. Optical element 344 may be subsequently coated with an anti-reflective dielectric stack to minimize light loss. Though the arrangement depicted has optical element 344 on the same side of substrate 320 as waveguide 334, waveguide 334 may be formed opposite optical element 344 with an opening in rigid beam input/output section 322 to permit radiation to pass through substrate 320.

A monolithic holder 350 includes recesses 352 to physically retain and guide rigid beam input/output sections 322. The cross-sectional view in FIG. 3F illustrates how substrate 320 may bend around holder 350. Holder 350 spatially fixes rigid beam input/output sections 322 about the tip of distal end 104, and flexible section 324 spans the portion of the holder between recesses 352. Although two rigid beam input/output sections 322 are shown in detail, each of recess 352 would ordinarily have a corresponding rigid beam input/output section 322 therein. Additional grooves may be provided in holder 350 for flexible sections 324. Holder 350 may alternatively be formed as a frame instead of a monolithic element.

FIG. 3G shows another alternative arrangement that includes a focusing element, thereby alleviating the need for a focusing element in view port 302. Instead of a refractive element shown in FIG. 3F, the arrangement in FIG. 3G includes optical element 344 with a reflective coating 346. Reflective coating 346 may be formed on a parabolic surface of optical element 344 to focus or collimate light from waveguide 334. Optical element 344 and reflective coating 346 may be formed on rigid beam input/output section 322 as described above. Alternatively, reflective coating 346 may be formed on a parabolic surface formed within recess 352 of holder 350, such that no additional optical element is needed. Reflective coating 346 may be designed for on-axis or off-axis reflection. For on-axis reflection, a substantially annular opening may be formed in beam input/output section 322 to allow beam 342 to pass through substrate 320 and around planar reflector 340.

In addition, the features described above with respect to FIGs 3D-3G may be combined to provide multiple means of directing beam of radiation 342.

FIG. 4A illustrates a view of the outside of distal section 104, according to an embodiment. Plurality of view ports 302 may be located anywhere around the entire outer surface of distal section 104 to provide any number of angles for viewing a tissue sample (e.g., an atrial wall) around distal section 104 and ablating a portion of the tissue sample by laser ablation. Additionally, distal section 104 may include a plurality of openings 402 that are associated with irrigation channels 310 shown in FIGs. 3A and 3B. Openings 402 may also be placed anywhere around the outer surface of distal section 104 and used to either expel liquid to the area surrounding distal section 104, or to draw liquid from the area surrounding distal section 104. In an embodiment, the plurality of view ports 302 and openings 402 may be placed at the same positions around the outer surface of distal section 104.

FIG. 4B illustrates an exploded view of distal section 104, according to an embodiment. Plurality of optical elements 404 may be located anywhere around the entire outer surface of a cap 410 to provide any number of angles for viewing a tissue sample (e.g., an atrial wall) around distal section 104 and ablating a portion of the tissue sample by laser ablation. Cap 410 is designed to fit around holder 350 and substrate 320. Rigid beam input/output sections 322 fit within recesses 352 of holder 350. Cap 410 then fits over holder 350 and substrate 320, and is secured by alignment and locking mechanisms 412 and 414. Detent 412 fits within intent 414 to secure cap 410 to holder 350 while permitting optical connection to proximal section 102. By securing cap 410 to holder 350, optical elements 404 are aligned with the optical output of rigid beam input/output sections 322.

In some embodiments, alignment between optical elements 404 and the optical outputs might not be needed for performing merged optical tissue evaluation and laser ablation. That is, cap 410 may include a material that is substantially transparent to a range of wavelengths of light used for optical interrogation and a range of wavelengths of light used for laser ablation. For example, the material of cap 410 may be substantially transparent at 1.3 $\mu$m for optical tissue evaluation and at 980 nm or 1060 nm for laser ablation. In some cases, cap 410 may include an anti-reflective coating to avoid undesired back reflections and provide a maximum transmitted energy to the tissue.

**Merged Optical Evaluation and Laser Ablation Embodiments**

FIGs. 5A and 5B illustrate example block diagrams of the merged optical evaluation and laser ablation system of the catheter, according to embodiments of the present disclosure. FIG. 5A illustrates an example system 500 with a first optical source 502, a second optical source 504, a first isolating element 503, a second isolating element 505, a coupling element 506, an optical splitter 508, a first multiplexer 510, a second multiplexer 512, and a coupling element 513. In some embodiments, each of the components of system 500 are housed within a catheter, such as catheter 100. For example, first optical source 502 and second optical source 504 may be located within proximal section 102 or processing device 108 of catheter 100. In some embodiments, first optical source 502 generates a source beam of radiation for optical evaluation of a sample 514 at a wavelength of about 1.3 $\mu$m, and second optical source 504 generates laser energy for tissue ablation of sample 514 at a wavelength of about 980 nm or about 1060 nm. First and second isolating elements 503 and 505 may include optical circulators or isolators that may be inserted in the catheter to avoid potential damage of first and second optical sources 502 and 504 by undesired back-reflections. The one or more exposure radiation beams and laser ablation energy beams generated by first and second optical sources 502 and 504 may be optically coupled by coupling element 506 in proximal section 102 of catheter 100. For example, coupling element 506 may be a fiber optic coupling element, in which the fiber optic coupling element allows first and second optical sources 502 and 504 to be coupled before being launched into the substrate of the catheter.

In some embodiments, coupling element 506 may couple first and second optical sources 502 and 504 together in a single fiber, for propagation of one or more exposure radiation beams and laser ablation energy beams in the same fiber down sheath 106 of catheter 100. Optical splitter 508 may separate or split the one or more exposure radiation beams from the one or more laser energy beams at the distal section 104 of catheter 100. In some embodiments, optical splitter 508 and/or coupling element 506 may be located in processing device 108 or proximal section 102 of catheter 100. After the beams are isolated, first multiplexer 510 may direct the one or more beams of exposure radiation to the plurality of optical ports 326 of distal section 104 of catheter 100. In some embodiments, first multiplexer 510 may include a passive multiplexer (e.g., distribution tree) or an active multiplexer (e.g., switch) in which the one or more beams of exposure may be directed to one or more optical ports 326. Second multiplexer 512 may direct the laser energy to at least one optical port of the plurality of optical ports 326 of distal section 104 of catheter 100. The one or more beams of exposure radiation and laser energy from first and second multiplexers 510 and 512 may be coupled by coupling element 513, which may be a fiber optic coupling element. The one or more beams of exposure radiation and laser ablation energy may then be transmitted to sample 514 from the plurality of optical ports 326. In some embodiments, first and second multiplexers 510 and 512 may be located in distal section 102 or proximal section 104 of catheter 100. In additional embodiments, a single multiplexer may be utilized to multiplex both the laser ablation energy and the one or more beams of exposure radiation to at least one optical port of the plurality of optical ports 326. Although FIG. 5A illustrates first and second multiplexers 510 and 512 with only one input and one output, first and second multiplexers 510 and 512 may comprise any number of inputs and any number of outputs in system 500.

FIG. 5B illustrates another example system 520 of a first optical source 522, a second optical source 524, a first isolating element 526, a second isolating element 528, a coupling element 530, and a multiplexer 532. In some embodiments, each of the components of system 520 are housed within a catheter, such as catheter 100. For example, first optical source 522 and second optical source 524 may be located within proximal section 102 or processing device 108 of catheter 100. In some embodiments, first and second optical sources 522 and 524 in FIG. 5B may be the same as first and second optical sources 502 and 504 in FIG. 5A. In system 520, one or more exposure radiation beams and laser ablation energy beams from first and second optical sources 522 and 524 may be propagated separately down the sheath 106 of catheter 100, such as in two separate fibers (e.g., one fiber for the one or more exposure radiation beams for optical evaluation of a sample 524, and another fiber for the one or more laser ablation energy beams for ablation of a portion of sample 524). First and second isolating elements 526 and 528 may include optical circulators or isolators that may be inserted in the catheter to avoid potential light source damage by undesired back-reflections. The one or more exposure radiation beams and laser ablation energy beams may be optically coupled by coupling element 530. In some embodiments, the coupling of the one or more exposure radiation beams and laser ablation energy beams occurs in distal section 104 of catheter 100. Multiplexer 532 may then direct the one or more beams of exposure radiation and laser energy to at least one optical port of the plurality of optical ports 326 of distal section 104 of catheter 100. In some embodiments, two multiplexers may be utilized to multiplex the laser energy and the one or more beams of exposure radiation separately to one or more optical ports of the plurality of optical ports 326. In additional embodiments, multiplexer 532 may be located in distal section 102 or proximal section 104 of catheter 100. Although FIG. 5B illustrates multiplexer 532 with only one input and one output, multiplexer 532 may comprise any number of inputs and any number of outputs in system 520.

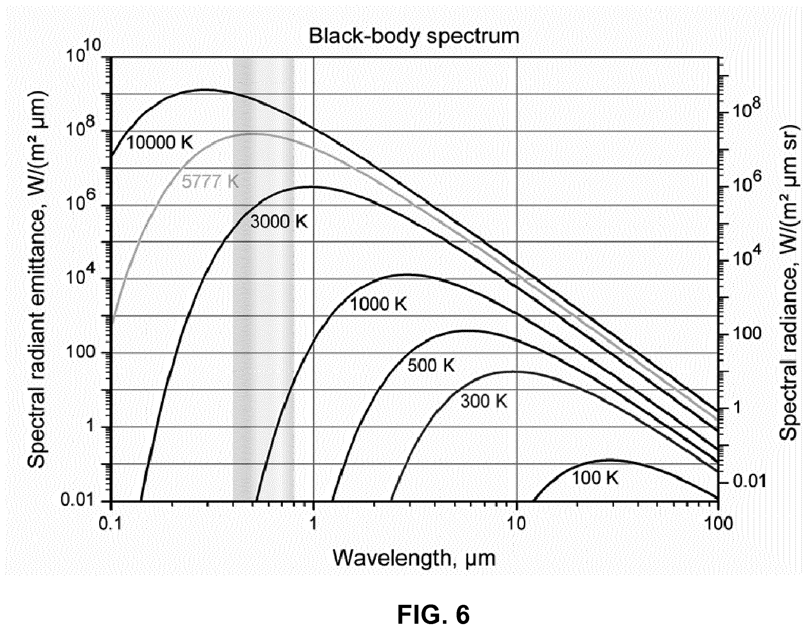

The merged optical evaluation and laser ablation catheter as described herein may be further configured to perform temperature measurements. For example, a detector (e.g., located in processing device 108 or in proximal section 102 or distal section 104 of catheter 100) may determine one or more temperatures of target tissue by measuring a blackbody spectrum emitted by the tissue before, during, or after laser ablation. FIG. 6 illustrates an example graph showing blackbody spectrum at different wavelengths and temperatures which may be measured by the ablation catheter, according to embodiments of the present disclosure. In order to measure temperatures in the range of human tissue temperatures (e.g., ranging from 37 °C to 80 °C during ablation), the detector of the catheter may be sensitive to wavelengths starting around 2 $\mu$m as shown in FIG. 6.

In some embodiments, the merged optical evaluation and laser ablation catheter may be implemented using silicon nitride as the material of the waveguide in order for low loss and broad bandwidth. Silicon nitride may allow optical waveguide loss as low as 0.7 dB/m, and the material may be transparent from 400 nm (silicon nitride bandgap) to 4000 nm ($SiO_2$ absorption) approximately. In some embodiments, silicon nitride materials may also have a higher refractive index than silicon dioxide materials as the wavelengths of interest (e.g., 980 nm for laser ablation, 1300 nm for optical tissue evaluation, 2000 nm for temperature measurements). Table 1 provides values of measured refractive index values of both $Si_3N_4$ and $SiO_2$ materials grown via Plasma-Enhanced Chemical Vapor Deposition (PECVD) as a function of the wavelength.

Table 1. Measured Refractive Index Values of $Si_3N_4$ and $SiO_2$

|  | 980 nm | 1300 nm | 2000 nm |
|---|---|---|---|
| $Si_3N_4$ | 1.8754 | 1.8729 | 1.8723 |
| $SiO_2$ | 1.4584 | 1.4576 | 1.4574 |

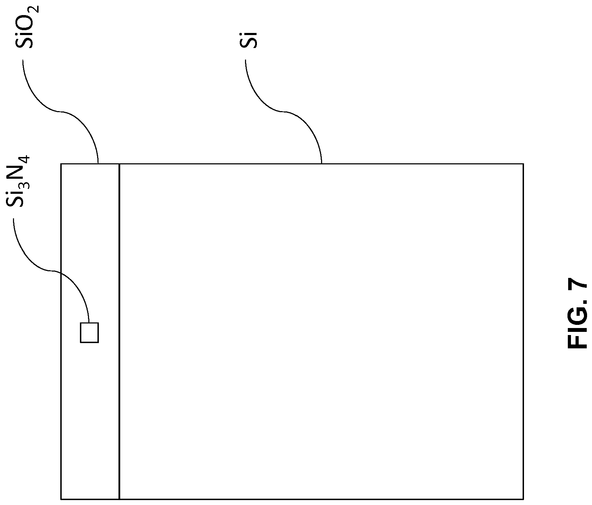

FIG. 7 illustrates an example diagram of a layer stack for a silicon nitride waveguide, according to embodiments of the present disclosure. The waveguide of FIG. 7 illustrates, for example, a 1-$\mu$m-width and 1-$\mu$m-height silicon nitride waveguide symmetrically embedded in a $SiO_2$ box. The embedded waveguide may be grown on a Si wafer to produce a photonic integrated circuit having rigid sections (e.g., rigid sections 322) joined by flexible sections (e.g., flexible sections 324) . In the flexible sections of the waveguide, the Si substrate may be removed from the stack of materials, among other possible changes.

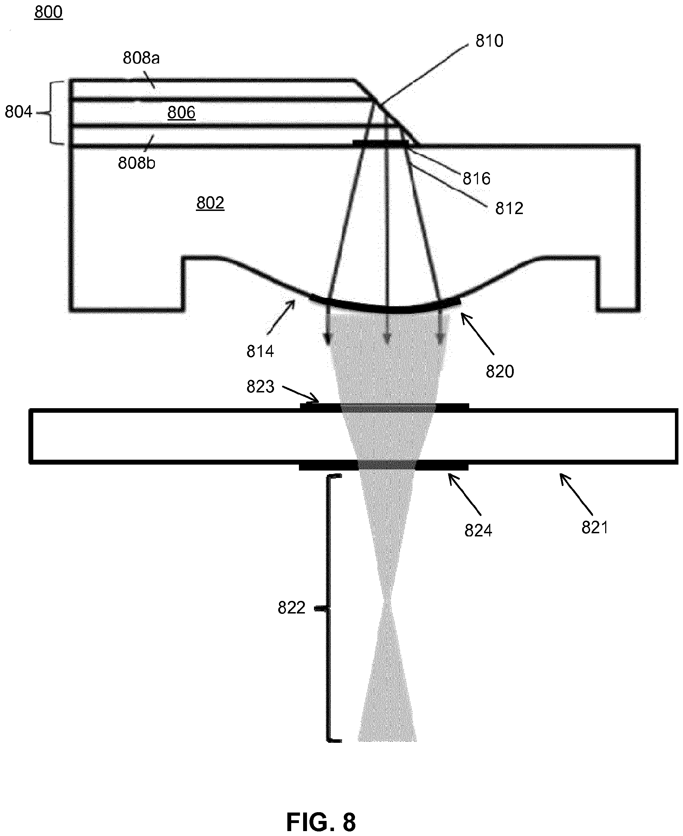

In some embodiments, the tissue evaluation functionality may be prioritized during design of the focusing optical elements of the substrate for the merged optical evaluation and laser ablation catheter. For example, a design wavelength of about 1300 nm, and reaching a depth of focus (DOF) of 1.5 mm in tissue, may be desired to accurately evaluate lesion transmurality. In order to get the desired DOF in tissue, a waist beam diameter of about 30.54 $\mu$m may be used at the focal point, which is placed at a distance equal to half DOF from the cap outer wall, e.g., 0.75 mm, according to an embodiment. FIG. 8 illustrates an example diagram of a device 800 designed to direct a beam of radiation, according to embodiments of the present disclosure. Device 800 includes a substrate 802, a waveguide 804 with a core layer 806 and cladding layers 808a and 808b. In an embodiment, substrate 802 may be silicon, core layer 806 may be silicon nitride, and cladding layers 808a and 808b may be silicon dioxide.

In an embodiment, reflector 810 is formed from a facet at the end of waveguide 804. In this way, a beam of radiation 812 is reflected downwards towards substrate 802 before it has exited from waveguide 804. An antireflective (AR) coating 816 may be included at an interface between waveguide 804 and substrate 802, according to an embodiment. AR coating 816 may be patterned such that it only exists beneath reflector 810. In another example, AR coating 816 covers a larger area on the surface of substrate 802. AR coating 816 may exist across the entire surface of substrate 802. FIG. 8 further illustrates an optical element 814 (e.g., a lens, mirror, or the like), a cap 821, a depth of focus (DOF) 822, and antireflective (AR) coatings 823 and 824. In one example, the refractive index of cladding layer 808b (no), AR coating layer 816, which may comprise silicon nitride or a more suitable material ($n_1$), and substrate 802 ($n_2$) are 1.4576, 1.8729 (if silicon nitride is considered) and 3.5226 at a wavelength of 1300 nm, respectively. In some embodiments, the radius of curvature of optical element 814 and the thickness of substrate 802 may be designed to result in a desired waist beam diameter of about 30.54 $\mu$m at the focal plane. The focal plane may be at half the depth of focus 822 and may correspond to the plane where the beam size is minimum.

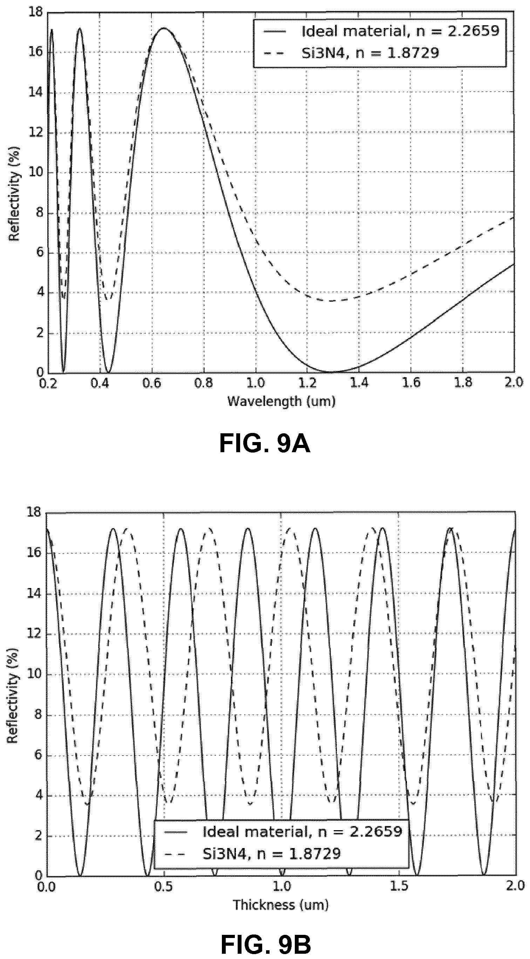

FIGs. 9A and 9B illustrate example graphs showing reflectivity as a function of wavelength and layer thickness, respectively, according to embodiments of the present disclosure. As shown in FIG. 9A, the reflectivity of substrate 802 with AR coating 816 is optimized at 1300 nm. The ideal material resulting in null reflectivity consists of a layer whose thickness is about 144 nm and refractive index $n_1$ = 2.2659. Apart from the ideal material candidate, the performance of having silicon nitride as an AR layer 816 is also considered. Under this scenario, the minimum reflectivity reaches about 3.5% when implementing a silicon nitride layer thickness of 174 nm. Not having any AR layer (e.g., AR coating 816) would result in a reflectivity of 9.34% at 1300 nm. By implementing an AR coating 816 at the interface between waveguide 804 and substrate 802, reflectivity from the substrate may be reduced in order for improved performance of the system.

In some embodiments, in order to avoid undesired back-reflections coming from the lens interface, an AR layer 820 is provided on the lenses, as shown in FIG. 8. In some embodiments, AR layer 820 may be designed to account for having saline (or any other fluid used in ablation procedures) in an irrigated catheter. For example, a refractive index change caused by saline or other fluid may necessitate a change in the refractive index and thickness of AR layer 820 to ensure reduced reflections, and AR layer 820 may be designed accordingly to account for these changes. In some embodiments, in non-irrigated approaches, AR layer 820 may be designed to account for having air in the lens surroundings. For example, a refractive index change caused by air in a non-irrigated catheter may necessitate a change in the refractive index and thickness of the AR layer 820 to ensure reduced reflections. In some embodiments, other irrigation-based approaches in which the irrigation holes in the cap (e.g., cap 410) are not aligned with the lenses may also be feasible.

In additional embodiments, AR layer 820 comprises silicon nitride. In some embodiments, a layer thickness of about 174 nm for AR layer 820 results in negligible reflectivity if used in air, while a reflectivity value of about 2% is reached when saline is in contact with the lenses. Similarly, AR layers 823 and 824 may similarly be designed to avoid undesired reflectivity in both the cap's 821 inner and outer surfaces.

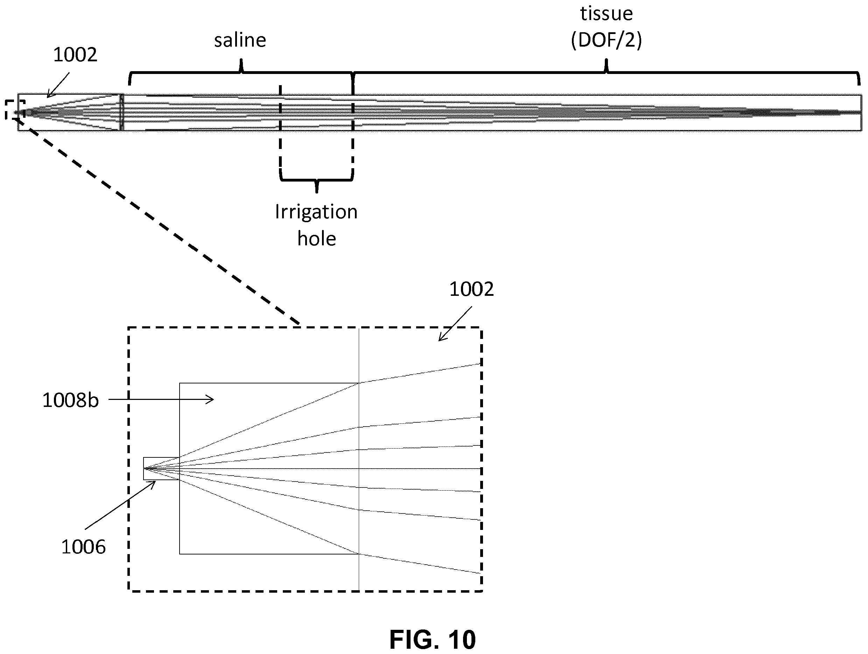

FIG. 10 illustrates an example environment of a waveguide with an irrigation approach, according to embodiments of the present disclosure. FIG. 10 shows a ray-tracing-based simulation that corresponds to an irrigated approach using the commercially available design software OpticStudio by ZEMAX LLC. In this example, FIG. 10 shows a silicon nitride waveguide with a core layer 1006 having a 1 $\mu$m x 1 $\mu$m cross-section, a silicon dioxide layer 1008b with a 5 $\mu$m thickness, and a silicon substrate material 1002. In some embodiments, core layer 1006, silicon dioxide layer 1008b, and substrate 1002 represent exemplary embodiments of core layer 806, cladding layers 808b, and substrate 802 in FIG. 8.

In some embodiments, the waveguide of FIG. 10 may include a lens (e.g., optical element 814), and the radius of curvature of the optical element and the thickness of substrate 1002 may be designed to result in a desired waist beam diameter of about 30.54 $\mu$m at the focal plane. In some cases, the focal plane may be at a distance of DOF/2 from the outer wall of the cap (e.g., cap 821). For example, the distance between the lens and the outer wall of the cap may be 0.5 mm, which includes the cap thickness, and the total distance between the lens and the focal plane may be fixed and equal to 1.25 mm. In an embodiment, an optimum radius of a lens (e.g., optical element 814) and a thickness of a substrate (e.g., substrate 1002) may be 116 $\mu$m and 181 $\mu$m, respectively. For example, these design parameters may result in a waist beam diameter at the focal plane of 28.82 $\mu$m.

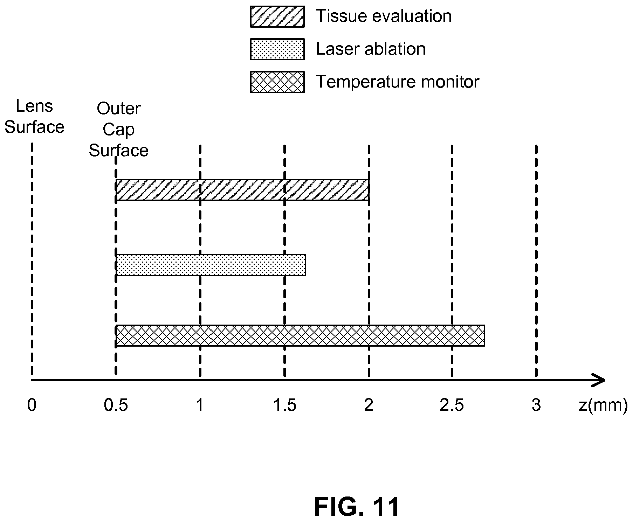

FIG. 11 illustrates an example graph showing focal plane positions of each wavelength at corresponding depth of focus (DOF) values, according to embodiments of the present disclosure. In some embodiments, the DOF values for the tissue evaluation and the temperature monitoring exceeds the DOF value corresponding to the laser ablation, resulting in accurate evaluation of the laser ablation procedure. In particular, tissue evaluation and temperature monitoring may occur at deeper depths compared to the DOF for the laser ablation, in order to keep the procedure under control and maintain safety of the patient during the procedure.

**High Power Considerations of Catheter Embodiments**

In some embodiments, there may be high power considerations for the merged optical evaluation and laser ablation system of the catheter. For example, the power of the laser ablation energy that is utilized to ablate tissue is in the order of few tens of Watts. The light-matter interaction may become non-linear for high optical intensities. In one embodiment, laser ablation energy emitted at a wavelength of 980 nm with powers up to 12W may be delivered to the target tissue. For example, in an embodiment of an embedded silicon nitride waveguide of 1 $\mu$m$^2$ cross-section area, the power density reaches about 1.2 GW/cm$^2$. In this embodiment, several non-linear effects may become relevant due to the relatively large optical intensities propagating through the waveguide, such as Two-Photon Absorption (TPA), nonlinear loss and refractive index, Four-Wave Mixing (FWM), Self-Phase Modulation (SPM) and Cross-Phase Modulation (XPM). In the presence of non-linear loss, the total loss coefficient may be described in Equation 1 as:

$$\alpha = \alpha_L + \alpha_{NL}(|E|^2) \qquad (1)$$

where $\alpha_L$, $\alpha_{NL}$ and $|E|^2$ accounts for the linear loss contributions, non-linear loss contributions, and the optical intensity, respectively.

During the TPA process, the band gap energy of a certain material may be bridged by the energy absorption of two photons, thus exciting electrons from the valence to the conduction bands. This process may induce non-linear loss $\alpha_{NL,TPA}$, which is dependent on the optical intensity according to Equation 2:

$$\alpha_{NL,TPA} = \alpha_2(|E|^2) \qquad (2)$$

where $\alpha_2$ is the TPA coefficient. The energy band gap of silicon nitride may be calculated as follows in Equation 3:

$$E_{g(SiN)} = h \cdot \frac{c_0 \cdot n_0}{\lambda_0} \cong 6\,eV \qquad (3)$$

where h, $c$, n and $\lambda_0$ are the Planck constant (h = 4.136·10$^{-15}$ $eV \cdot s$), the speed of light in the vacuum, the refraction index of silicon nitride ($n_0$ = 1.9378) and the onset wavelength, which is about 400 nm, respectively. On the other hand, the energy band gap at the laser ablation wavelength (e.g., at 980 nm), is calculated by Equation 4:

$$E_{g,980} = h \cdot \frac{c_0 \cdot n_{980}}{\lambda_{980}} \cong 2.37\,eV \qquad (4)$$

Equations 3 and 4 show that TPA should not occur in silicon nitride at 980 nm because photon energies at this wavelength are less than $E_{g(siN)}/2$. In addition, an optical intensity of 1.2 GW/cm$^2$ assures a linear regime in the waveguide because non-linear loss is induced by power densities typically at least one order of magnitude higher.

In some embodiments, high optical intensities may also lead to non-linear refractive index, in which the dependence can be written as shown in Equation 5:

$$n(\omega, |E|^2) = n_0(\omega) + n_2|E|^2 \tag{5}$$

where E is the electric field amplitude and $n_2$ is the non-linear Kerr coefficient that is related to the third-order material susceptibility $\chi^{(3)}$ by Equation 6

$$n_2 = \frac{3}{4}\frac{\chi^{(3)}}{c\varepsilon_0^2} \tag{6}$$

In an example, the non-linear Kerr coefficient was experimentally measured in silicon nitride waveguides to be $n_2 = 2.4 \cdot 10^{-15}$ cm$^2$/W. This measurement indicates refractive index changes in the order of $2.88 \cdot 10^{-6}$ at 980 nm, which is more than one order of magnitude lower compared with the thermo-optical effect efficiency in silicon nitride.

In $\chi^{(3)}$-materials, the third-order polarization term may involve the nonlinear interaction of four waves and leads to the phenomenon of FWM. In an embodiment, the FWM may result from the radiation-induced modulation of the refractive index as shown by Equation 5, where E(t) = $E_1\cos(2\pi f_1 t) + E_2\cos(2\pi f_2 t) + E_3\cos(2\pi f_3 t)$ with $f_1 = 0.15 \cdot 10^{15}$ Hz, $f_2 = 0.231 \cdot 10^{15}$ Hz, and fa = $0.306 \cdot 10^{15}$ Hz. The frequencies $f_1$, $f_2$, and $f_3$ correspond to the frequencies of the temperature monitoring, the tissue evaluation, and the ablation systems respectively. As a result, the generation of light at the new frequencies are detailed in the following Table 2:

Table 2. Generated Frequency Values

| Generated Frequency | Value (Hz) | Value (nm) |
|---|---|---|
| $f_1 + f_2 - f_3$ | $74.67 \cdot 10^{12}$ | 4018.92 |
| $f_1 + f_3 - f_2$ | $225.35 \cdot 10^{12}$ | 1331.24 |
| $f_2 + f_3 - f_1$ | $386.89 \cdot 10^{12}$ | 775.41 |
| $2f_1 + f_2$ | $530.77 \cdot 10^{12}$ | 565.22 |
| $2f_1 - f_2$ | $69.23 \cdot 10^{12}$ | 4333.33 |
| $2f_1 + f_3$ | $606.12 \cdot 10^{12}$ | 494.95 |
| $2f_1 - f_3$ | - | - |
| $2f_2 + f_1$ | $611.54 \cdot 10^{12}$ | 490.57 |
| $2f_2 - f_1$ | $311.54 \cdot 10^{12}$ | 962.96 |
| $2f_2 + f_3$ | $767.66 \cdot 10^{12}$ | 390.80 |
| $2f_2 - f_3$ | $155.42 \cdot 10^{12}$ | 1930.30 |
| $2f_3 + f_1$ | $762.24 \cdot 10^{12}$ | 393.57 |
| $2f_3 - f_1$ | $462.24 \cdot 10^{12}$ | 649.01 |
| $2f_3 + f_2$ | $843.01 \cdot 10^{12}$ | 355.87 |
| $2f_3 - f_2$ | $381.48 \cdot 10^{12}$ | 786.42 |
| $3f_1$ | $450 \cdot 10^{12}$ | 666.66 |
| $3f_2$ | $692.31 \cdot 10^{12}$ | 433.33 |
| $3f_3$ | $918.37 \cdot 10^{12}$ | 326.66 |
| $f_1 + f_2 + f_3$ | $686.89 \cdot 10^{12}$ | 436.75 |

In some embodiments, the new frequencies that are below 400 nm and greater than 4000 nm in Table 2 may not be propagated due to the silicon nitride bandgap and $SiO_2$ absorption, respectively. Moreover, the generated frequencies that involve the temperature signal ($f_1$) are expected to be negligible because of its very low optical intensity (e.g., according to the black body spectrum shown in FIG. 6). Therefore, the generated new frequencies with relevant intensity values are those centered at $2f_2 - f_3$, $2f_3 - f_2$ and $3f_2$, with respect to the tissue evaluation and temperature monitoring operating wavelength range. In some embodiments, these wavelengths (1930 nm, 786 nm, and 433 nm) may not interfere with the laser ablation wavelength (980 nm), the optical evaluation wavelengths (1250 - 1350 nm), nor the temperature measurement wavelength (> 2000 nm). In additional embodiments, an optical filter may be applied before a detector measures the temperature of target tissue (e.g., at 2000 nm wavelength) in order to filter out wavelengths lower than a predetermined wavelength (e.g., 1950 nm). By utilizing one or more optical filters, the system may avoid false temperature measurements of target tissue.

In some embodiments, another consequence of the Kerr effect may be Self-Phase Modulation (SPM). As derived by Equation 5, relatively large optical intensities may result in a varying refractive index of the medium enabled by light-matter interaction. This variation in the refractive index produces a phase shift of the optical signal, which translates into a broadening of the frequency spectrum. In some embodiments, this change may be irrelevant for both the tissue evaluation and the temperature monitoring systems because the change in the spectrum characteristics of the laser ablation signal may not have any impact on them. However, apart from SPM, Cross-Phase Modulation (XPM) may also occur. XPM is similar to SPM, but the phase shift induced by the intensity of light affects other frequencies instead of the laser ablation frequency. The strength of the XPM effect decreases with the wavelength detuning between the pump (laser ablation) and the probe (tissue evaluation) signals. Taking into account that the wavelength detuning may be greater than 300 nm, this effect might not provide any significant impact on the tissue evaluation wavelengths.

**Example Method of Operation**

Catheter 100 may be used to perform merged optical evaluation, laser ablation, and temperature monitoring of target tissue according to embodiments described herein. At least a portion of tissue volume surrounding one or more optical ports of the plurality of optical ports in the distal section 104 of catheter 100 may be ablated. By adjusting the laser power and ablation time, the total laser energy delivered to target tissue may be accurately controlled. In additional embodiments, the catheter may also provide temperature monitoring and additional cooling to surrounding blood flow around the target tissue.

Various ablation methods and other embodiments of ablation catheters with substrates described thus far can be implemented, for example, using catheter 100 shown in FIG. 1, distal section 104 as shown in FIGs. 3A-3G and 4A-4B, and the merged optical evaluation and laser ablation system shown in FIGs. 5A-5B, and the embodiments shown in FIGs. 6-11.

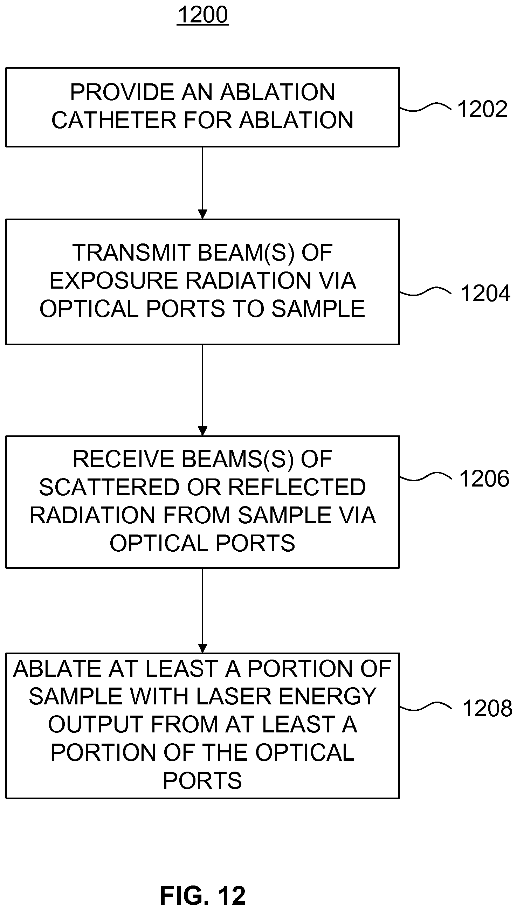

FIG. 12 illustrates an example method 1200 for performing merged optical tissue evaluation and laser ablation according to embodiments of the present disclosure. Method 1200 may be performed by ablation catheter 100 as described herein.

At block 1202, an ablation catheter is provided. For example, an ablation catheter with a proximal end, a distal end with a plurality of optical ports, and a sheath coupled between the proximal end and the distal end is provided. For example, the ablation catheter may include a holder configured to maintain the plurality of optical ports in a fixed spatial relationship and a cap that is substantially transparent at wavelengths of the exposure radiation, the temperature monitoring system and laser energy, in which the cap is secured to the holder and configured to cover the holder and the plurality of optical ports. At block 1204, one or more beams of exposure radiation may be transmitted to a sample via the plurality of optical ports. For example, the one or more beams of exposure radiation may be provided from a first optical source configured to generate a source beam of exposure radiation. The one or more beams of exposure radiation from the source beam may be directed to the plurality of optical ports by a multiplexer located in the proximal end or distal end of the catheter.

At block 1206, one or more beams of scattered or reflected radiation from the sample may be received from the sample via the plurality of optical ports. For example, the one or more beams of scattered or reflected radiation may be guided by optical transmission media in the sheath of the catheter

At block 1208, at least a portion of the sample may be ablated with laser energy output from at least a portion of the optical ports. For example, the laser energy may be provided from a second optical source configured to generate the laser energy. The laser energy from the second optical source may be directed to at least one optical port by a multiplexer located in the proximal end or distal end of the catheter.

It is to be appreciated that the Detailed Description section, and not the Summary and Abstract sections, is intended to be used to interpret the claims. The Summary and Abstract sections may set forth one or more but not all exemplary embodiments of the present invention as contemplated by the inventor(s), and thus, are not intended to limit the present invention and the appended claims in any way.

Embodiments of the present invention have been described above with the aid of functional building blocks illustrating the implementation of specified functions and relationships thereof. The boundaries of these functional building blocks have been arbitrarily defined herein for the convenience of the description. Alternate boundaries can be defined so long as the specified functions and relationships thereof are appropriately performed.

The foregoing description of the specific embodiments will so fully reveal the general nature of the invention that others can, by applying knowledge within the skill of the art, readily modify and/or adapt for various applications such specific embodiments, without undue experimentation, without departing from the general concept of the present invention. Therefore, such adaptations and modifications are intended to be within the meaning and range of the disclosed embodiments, based on the teaching and guidance presented herein. It is to be understood that the phraseology or terminology herein is for the purpose of description and not of limitation, such that the terminology or phraseology of the present specification is to be interpreted by the skilled artisan in light of the teachings and guidance.

The breadth and scope of the present invention should not be limited by any of the above-described exemplary embodiments, but should be defined only in accordance with the following claims.

**Claims**

1. A catheter system comprising:

 a processing device (108) comprising a first optical source (502) configured to generate a source beam of exposure radiation, a second optical source (504) configured to generate the laser ablation energy, and a multiplexer; and a catheter (100) comprising:

 a proximal section (102), a distal section (104) and a sheath (106) coupled between the proximal section (102) and the distal section (104);
 the distal section (104) comprising:
 a plurality of optical ports (326) configured to:

 transmit one or more beams of exposure radiation to a sample (308), receive one more or beams of scattered radiation that have been reflected or scattered from the sample (308), and transmit laser ablation energy such that at least a portion of the sample (308) is ablated, and
 a holder (350) configured to maintain the plurality of optical ports (326) in a fixed spatial relationship,

 wherein the multiplexer is configured to:

 direct the one or more beams of exposure radiation from the source beam of radiation to the plurality of optical ports (326),
 combine the one or more beams of scattered radiation; and
 direct the laser ablation energy to at least one optical port (326) of the plurality of optical ports (326),
 wherein the plurality of optical ports (326) are formed on a substrate having rigid sections and flexible sections.

2. The catheter system of claim 1, wherein the plurality of optical ports (326) are formed on the rigid sections of the substrate, wherein the rigid sections are interconnected by the flexible sections.

3. The catheter system of claim 1, wherein the first and second optical sources (502, 504) are optically coupled by a coupling element (506) in the distal end of the catheter (100).

4. The catheter system of claim 1, wherein the first and second optical sources (502, 504) are optically coupled by a coupling element (506) in the proximal end of the catheter (100).

5. The catheter system of claim 1, further comprising:
 a cap (410) that is substantially transparent at wavelengths of the one or more beams of exposure radiation, the one or more beams of scattered radiation, and the laser ablation energy, wherein the cap (410) is secured to the holder (350) and configured to cover the holder (350) and the plurality of optical ports (326).

6. The catheter system of claim 1, wherein the multiplexer is further configured to perform phase shifting.

7. The catheter system of claim 1, wherein the plurality of optical ports (326) is further configured to receive one or more signals indicating a temperature of the portion of the sample (308).

8. A catheter system for performing merged optical tissue evaluation and laser ablation, the catheter system comprising: a processing device (108), comprising a first optical source (502) configured to generate a source beam of exposure radiation and a second optical source (504) configured to generate the laser ablation energy, and a catheter (100) comprising:

a proximal section (102), a distal section (104) and a sheath (106) coupled between the proximal section (102) and the distal section (104);
the distal section (104) comprising:
a plurality of optical ports (326) configured to:

transmit one or more beams of exposure radiation to a sample (308),
receive one or more beams of scattered radiation that have been reflected or scattered from the sample (308), and
transmit laser ablation energy such at least a portion of the sample (308) is ablated;
wherein the plurality of optical ports (326) are formed on a substrate having rigid sections and flexible sections.

9. The catheter system of claim 8, the catheter (100) further comprising:
a holder (350) configured to maintain the plurality of optical ports (326) in a fixed spatial relationship.

10. The catheter system of claim 8, wherein the first and second optical sources (502, 504) are optically coupled by a coupling element (506) in the distal end or the proximal end of the catheter (100).

11. The catheter system of claim 8, further comprising:
a first multiplexer (510) configured to:

direct the one or more beams of exposure radiation from the source beam of radiation to the plurality of optical ports (326), and
combine the one or more beams of scattered radiation; and
a second multiplexer (512) configured to:
direct the laser ablation energy to at least one of the plurality of optical ports (326).

**Patentansprüche**

1. Kathetersystem, aufweisend:
eine Verarbeitungsvorrichtung (108) mit einer ersten optischen Quelle (502), die dafür ausgelegt ist, einen Quellenstrahl einer Belichtungsstrahlung zu erzeugen, einer zweiten optischen Quelle (504), die dafür ausgelegt ist, Laserablationsenergie zu erzeugen, und einen Multiplexer; und einen Katheter (100), aufweisend:

ein proximales Teilstück (102), ein distales Teilstück (104) und eine Umhüllung (106), die zwischen dem proximalen Teilstück (102) und dem distalen Teilstück (104) angeschlossen ist;
wobei das distale Teilstück (104) aufweist:
mehrere optische Öffnungen (326), die ausgelegt sind zum:

Übertragen von einem oder mehreren Strahlen der Belichtungsstrahlung auf eine Materialprobe (308),
Empfangen von einem oder mehreren Strahlen einer Streustrahlung, die von der Materialprobe (308) reflektiert oder gestreut wurden, und Übertragen der Laserablationsenergie in der Weise, dass mindestens ein Teilbereich der Materialprobe (308) abgetragen wird, und
einen Halter (350), der dafür ausgelegt ist, die mehreren optischen Öffnungen (326) in einer festen räumlichen Beziehung zu halten,
wobei der Multiplexer ausgelegt ist zum:

Richten des einen oder der mehreren Strahlen der Belichtungsstrahlung vom Quellenstrahl der Strahlung auf die mehreren optischen Öffnungen (326),

Zusammenführen des einen oder der mehreren Strahlen der Streustrahlung; und

Richten der Laserablationsenergie auf mindestens eine optische Öffnung (326) der mehreren optischen Öffnungen (326),

wobei die mehreren optischen Öffnungen (326) auf einem Trägermaterial mit starren Teilstücken und flexiblen Teilstücken gebildet sind.

2. Kathetersystem nach Anspruch 1, wobei die mehreren optischen Öffnungen (326) auf den starren Teilstücken des Trägermaterials gebildet sind, wobei die starren Teilstücke durch die flexiblen Teilstücke untereinander verbunden sind.

3. Kathetersystem nach Anspruch 1, wobei die erste und zweite optische Quelle (502, 504) durch ein Kopplungselement (506) im distalen Ende des Katheters (100) optisch gekoppelt sind.

4. Kathetersystem nach Anspruch 1, wobei die erste und zweite optische Quelle (502, 504) durch ein Kopplungselement (506) im proximalen Ende des Katheters (100) optisch gekoppelt sind.

5. Kathetersystem nach Anspruch 1, darüber hinaus aufweisend:

eine Kappe (410), die bei Wellenlängen des einen oder der mehreren Strahlen der Belichtungsstrahlung, des einen oder der mehreren Strahlen der Streustrahlung und der Laserablationsenergie im Wesentlichen transparent ist, wobei die Kappe (410) am Halter (350) befestigt und dafür ausgelegt ist, den Halter (350) und die mehreren optischen Öffnungen (326) abzudecken.

6. Kathetersystem nach Anspruch 1, wobei der Multiplexer darüber hinaus dafür ausgelegt ist, eine Phasenverschiebung durchzuführen.

7. Kathetersystem nach Anspruch 1, wobei die mehreren optischen Öffnungen (326) darüber hinaus dafür ausgelegt sind, ein oder mehrere Signale zu empfangen, die eine Temperatur des Teilbereichs der Materialprobe (308) angeben.

8. Kathetersystem zum Durchführen einer zusammengefassten optischen Gewebebeurteilung und Laserablation, wobei das Kathetersystem aufweist:

eine Verarbeitungsvorrichtung (108) mit einer ersten optischen Quelle (502), die dafür ausgelegt ist, einen Quellenstrahl einer Belichtungsstrahlung zu erzeugen, und einer zweiten optischen Quelle (504), die dafür ausgelegt ist, Laserablationsenergie zu erzeugen, und einen Katheter (100), aufweisend:

ein proximales Teilstück (102), ein distales Teilstück (104) und eine Umhüllung (106), die zwischen dem proximalen Teilstück (102) und dem distalen Teilstück (104) angeschlossen ist;
wobei das distale Teilstück (104) aufweist:
mehrere optische Öffnungen (326), die ausgelegt sind zum:

Übertragen von einem oder mehreren Strahlen einer Belichtungsstrahlung auf eine Materialprobe (308),
Empfangen von einem oder mehreren Strahlen einer Streustrahlung, die von der Materialprobe (308) reflektiert oder gestreut wurden, und
Übertragen der Laserablationsenergie in der Weise, dass mindestens ein Teilbereich der Materialprobe (308) abgetragen wird;
wobei die mehreren optischen Öffnungen (326) auf einem Trägermaterial mit starren Teilstücken und flexiblen Teilstücken gebildet sind.

9. Kathetersystem nach Anspruch 8, wobei der Katheter (100) darüber hinaus aufweist:
einen Halter (350), der dafür ausgelegt ist, die mehreren optischen Öffnungen (326) in einer festen räumlichen Beziehung zu halten.

10. Kathetersystem nach Anspruch 8, wobei die erste und zweite optische Quelle (502, 504) durch ein Kopplungselement (506) im distalen Ende oder proximalen Ende des Katheters (100) optisch gekoppelt sind.

11. Kathetersystem nach Anspruch 8, darüber hinaus aufweisend:
einen ersten Multiplexer (510), der ausgelegt ist zum:

Richten des einen oder der mehreren Strahlen der Belichtungsstrahlung vom Quellenstrahl der Strahlung auf die mehreren optischen Öffnungen (326), und

Zusammenführen des einen oder der mehreren Strahlen der Streustrahlung; und

einen zweiten Multiplexer (512), der ausgelegt ist zum:

Richten der Laserablationsenergie auf mindestens eine der mehreren optischen Öffnungen (326).

**Revendications**

1. Système de cathéter, comprenant :

   un dispositif de traitement (108) comprenant une première source optique (502) configurée pour générer un faisceau source de rayonnement d'exposition, une seconde source optique (504) configurée pour générer l'énergie d'ablation par laser et un multiplexeur ; et

   un cathéter (100) comprenant :

   une section proximale (102), une section distale (104) et une gaine (106) couplée entre la section proximale (102) et la section distale (104) ;
   la section distale (104) comprenant :
   une pluralité de ports optiques (326) configurés pour :

   transmettre un ou plusieurs faisceaux de rayonnement d'exposition à un échantillon (308), recevoir un ou

   plusieurs faisceaux de rayonnement diffusé qui ont été réfléchis ou dispersés à partir de l'échantillon (308) et

   transmettre l'énergie d'ablation par laser de telle sorte qu'au moins une partie de l'échantillon (308) soit ablatée ; et

   un support (350) configuré pour maintenir la pluralité de ports optiques (326) dans une relation spatiale fixe ;
   dans lequel le multiplexeur est configuré pour :

   diriger le ou les faisceaux de rayonnement d'exposition depuis le faisceau de rayonnement source jusqu'à la pluralité de ports optiques (326) ;
   combiner le ou les faisceaux de rayonnement diffusé ; et
   diriger l'énergie d'ablation par laser vers au moins un port optique (326) de la pluralité de ports optiques (326),
   dans lequel la pluralité de ports optiques (326) sont formés sur un substrat ayant des sections rigides et des sections flexibles.

2. Système de cathéter selon la revendication 1, dans lequel la pluralité de ports optiques (326) sont formés sur les sections rigides du substrat, dans lequel les sections rigides sont interconnectées par les sections flexibles.

3. Système de cathéter selon la revendication 1, dans lequel les première et seconde sources optiques (502, 504) sont couplées sur le plan optique par un élément de couplage (506) situé dans l'extrémité distale du cathéter (100) .

4. Système de cathéter selon la revendication 1, dans lequel les première et seconde sources optiques (502, 504) sont couplées sur le plan optique par un élément de couplage (506) situé dans l'extrémité proximale du cathéter (100) .

5. Système de cathéter selon la revendication 1, comprenant en outre :
   un cache (410) qui est sensiblement transparent aux longueurs d'onde d'un ou de plusieurs faisceaux de rayonnement d'exposition, du ou des faisceaux de rayonnement diffusé et de l'énergie d'ablation par laser, dans lequel le cache (410) est fixé de façon sécurisée au support (350) et configuré pour recouvrir le support (350) et la pluralité de ports optiques (326).

6. Système de cathéter selon la revendication 1, dans lequel le multiplexeur est en outre configuré pour réaliser un décalage de phase.

7. Système de cathéter selon la revendication 1, dans lequel la pluralité de ports optiques (326) est en outre configurée pour recevoir un ou plusieurs signaux indiquant une température de la partie de l'échantillon (308).

8. Système de cathéter conçu pour réaliser une évaluation de tissu optique fusionné et une ablation par laser, le système de cathéter comprenant :

un dispositif de traitement (108), comprenant une première source optique (502) configurée pour générer un faisceau source de rayonnement d'exposition et une seconde source optique (504) configurée pour générer l'énergie d'ablation par laser ; et
un cathéter (100) comprenant :

une section proximale (102), une section distale (104) et une gaine (106) couplée entre la section proximale (102) et la section distale (104) ;
la section distale (104) comprenant :
une pluralité de ports optiques (326) configurés pour :

transmettre un ou plusieurs faisceaux de rayonnement d'exposition à un échantillon (308) ;
recevoir un ou plusieurs faisceaux de rayonnement diffusé qui ont été réfléchis ou dispersés à partir de l'échantillon (308) ; et
transmettre l'énergie d'ablation par laser de façon à ce qu'au moins une partie de l'échantillon (308) soit ablatée ;
dans lequel la pluralité de ports optiques (326) sont formés sur un substrat ayant des sections rigides et des sections flexibles.

9. Système de cathéter selon la revendication 8, le cathéter (100) comprenant en outre :
un support (350) configuré pour maintenir la pluralité de ports optiques (326) dans une relation spatiale fixe.

10. Système de cathéter selon la revendication 8, dans lequel les première et seconde sources optiques (502, 504) sont couplées sur le plan optique au moyen d'un élément de couplage (506) situé dans l'extrémité distale ou l'extrémité proximale du cathéter (100).

11. Système de cathéter selon la revendication 8, comprenant en outre :
un premier multiplexeur (510) configuré pour :

diriger le ou les faisceaux de rayonnement d'exposition du faisceau de rayonnement source jusqu'à la pluralité de ports optiques (326) ; et
combiner le ou les faisceaux de rayonnement diffusé ; et
un second multiplexeur (512) configuré pour :
diriger l'énergie d'ablation par laser vers au moins un port parmi la pluralité de ports optiques (326).

**FIG. 1**

**FIG. 2A**

**FIG. 2B**

**FIG. 3A**

**FIG. 3B**

**FIG. 3C**

EP 3 806 770 B1

**FIG. 3D**

**FIG. 3E**

FIG. 3F

352

350

322

334

324

344

342

FIG. 3G

FIG. 4A

FIG. 4B

**FIG. 5A**

**FIG. 5B**

**FIG. 6**

**FIG. 7**

EP 3 806 770 B1

FIG. 8

**FIG. 9A**

**FIG. 9B**

FIG. 10

**FIG. 11**

<u>1200</u>

FIG. 12

**REFERENCES CITED IN THE DESCRIPTION**

**Patent documents cited in the description**

- WO 2016187664 A1
- US 9062960 B
- US 9354040 B
- US 9690093 B